# EUROPEAN PATENT SPECIFICATION

(11) **EP 1 117 401 B1**
(45) Date of publication and mention of the grant of the patent: **19.11.2003**
(21) Application number: 99956504.7
(22) Date of filing: 29.09.1999
(51) Int. Cl.: A61K 31/435, A61K 31/47, A61K 31/535, A61K 31/57, A61P 27/02, A61P 27/16, A61K 45/06

(54) **ANTIBIOTIC COMPOSITIONS FOR TREATMENT OF THE EYE**
ANTIBIOTISCHE ZUSAMMENSETZUNGEN ZUR BEHANDLUNG DES AUGES
COMPOSITIONS ANTIBIOTIQUES POUR LE TRAITEMENT DES YEUX

(30) Priority: 30.09.1998 US 102504 P; 30.09.1998 US 102506 P
(43) Date of publication of application: 25.07.2001
(60) Divisional application: 03020587.6
(73) Proprietor: Alcon, Inc., 6331 Hünenberg (CH)
(72) Inventor: CAGLE, Gerald, Fort Worth, TX 76116 (US); ABSHIRE, Robert, L., Fort Worth, TX 76116 (US); STROMAN, David, W., Irving, TX 75063 (US); YANNI, John, M., Burleson, TX 76028 (US)
(74) Representative: Best, Michael, Dr.
(86) International application number: US9922622
(87) International publication number: WO00018386

(56) References cited:
- EP-A- 0 550 903
- WO-A-90/01933
- WO-A-96/39146
- WO-A-99/15172
- DE-A- 4 424 369
- US-A- 4 990 517
- US-A- 5 223 493
- US-A- 5 607 942
- KRASEMANN, C. (1) ET AL: "Efficacy of moxifloxacin against Staphylococcus aureus in respiratory tract and skin and skin structure infections." JOURNAL OF ANTIMICROBIAL CHEMOTHERAPY, (JULY, 1999) VOL. 44, NO. SUPPL. A, PP. 150. MEETING INFO.: 21ST INTERNATIONAL CONGRESS OF CHEMOTHERAPY BIRMINGHAM, ENGLAND, UK JULY 4-7, 1999 , XP000892776
- ELIES W.: "[Novel fluoroquinolones in the treatment of ENT infections]. NEUERE FLUORCHINOLONE BEI DER THERAPIE VON HNO-INFEKTIONEN." CHEMOTHERAPIE JOURNAL, (1998) 7/3 (93-97). , XP000892813

## Description

### Backeround of the Invention

The present invention is directed to the provision of topical antibiotic pharmaceutical compositions for the treatment of ophthalmic infections, particularly bacterial infections. These compositions can be used to treat ophthalmic infections by applying those compositions to the affected tissues. The compositions and methods of the invention are based on the use of a new antibiotic. The compositions of the present invention may also contain one or more anti-inflammatory agents.

The use of quinolone antibiotics to treat infections represents the current state of the art in the field of ophthalmic pharmaceutical compositions and methods of treatment. For example, a topical ophthalmic composition containing the quinolone ciprofloxacin is marketed by Alcon Laboratories, Inc. under the name CILOXAN™ (Ciprofloxacin 0.3%) Ophthalmic Solution. The following quinolones have also been utilized in ophthalmic antibiotic compositions:

| **Quinolone** | **Product** | **Manufacturer** |
|---|---|---|
| Ofloxacin | OCUFLOX™ | Allergan |
| Norfloxacin | CHIBROXIN™ | Merck |
| Lomefloxacin | LOMEFLOX™ | Senju |

The foregoing quinolone antibiotic compositions are generally effective in treating ophthalmic infections, and have distinct advantages over prior ophthalmic antibiotic compositions, particularly those having relatively limited spectrums of antimicrobial activity, such as: neomycin, polymyxin B, gentamicin and tobramycin, which are primarily useful against gram negative pathogens; and bacitracin, gramicidin, and erythromycin, which are primarily active against gram positive pathogens. However, despite the general efficacy of the ophthalmic quinolone therapies currently available, there is a need for improved compositions and methods of treatment based on the use of antibiotics that are more effective than existing antibiotics against key ophthalmic pathogens, and less prone to the development of resistance by those pathogens.

Ophthalmic infections are frequently accompanied by inflammation of the infected ophthalmic tissues and perhaps even surrounding tissues. Similarly, ophthalmic surgical procedures that create a risk of microbial infections frequently also cause inflammation of the affected tissues. Thus, there is also a need for ophthalmic pharmaceutical compositions that combine the anti-infective activity of one or more antibiotics with the anti-inflammatory activity of one or more steroid or non-steroid agents in a single composition.

### Summary of the Invention

The invention is based on the use of a potent new antibiotic to treat ophthalmic infections, as well as the prophylactic use of this antibiotic following surgery or other trauma to ophthalmic tissues. The compositions of the present invention may also be administered to the affected tissues during ophthalmic surgical procedures to prevent or alleviate post-surgical infection.

The compositions preferably also contain one or more anti-inflammatory agents to treat inflammation associated with infections of ophthalmic tissues. The anti-inflammatory component of the compositions is also useful in treating inflammation associated with physical trauma to ophthalmic tissues, including inflammation resulting from surgical procedures. The compositions of the present invention are therefore particularly useful in treating inflammation associated with trauma to ophthalmic tissues wherein there is either an infection or a risk of an infection resulting from the trauma.

Examples of ophthalmic conditions that may be treated with the compositions of the present invention include conjunctivitis, keratitis, blepharitis, dacyrocystitis, hordeolum and corneal ulcers. The compositions of the invention may also be used prophylactically in connection with various ophthalmic surgical procedures that create a risk of infection.

The compositions of the present invention are specially formulated for topical application to ophthalmic tissues. The compositions are preferably sterile, and have physical properties (e.g., osmolality and pH) that are specially suited for application to ophthalmic tissues, including tissues that have been compromised as the result of preexisting disease, trauma, surgery or other physical conditions.

### Detailed Description of the Invention

The antibiotic used in the compositions and methods of the present invention is Moxifloxacin or a pharmaceutically useful hydrate or salf thereof. Moxifloxacin has the following structure: 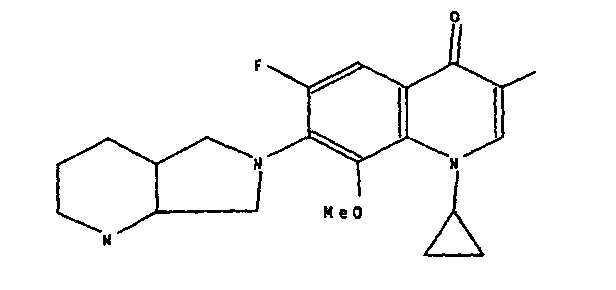 Further details regarding the structure, preparation, and physical properties of Moxifloxacin are provided in United States Patent No. 5,607,942.

The concentrations of Moxifloxacin or a pharmaceutically useful hydrate or salt thereof in the compositions of the present invention is 0.1 to 1.0 wt.% and will vary depending on the intended use of the compositions (e.g., treatment of existing infections or prevention of post-surgical infections). The antimicrobial activity of antibiotics is generally expressed as the minimum concentration required to inhibit the growth of a specified pathogen. This concentration is also referred to as the "minimum inhibitory concentration" or "MIC". The term "MIC90" refers to the minimum concentration of antibiotic required to inhibit the growth of ninety percent (90%) of the strains of a species. The concentration of an antibiotic required to totally kill a specified bacteria is referred to as the "minimum bactericidal concentration" or "MBC". The minimum inhibitory concentration of Moxifloxacin for several bacteria commonly associated with ophthalmic infections are provided in the following table:

| Microorganism | MIC₉₀ |
|---|---|
| S. aureus/methicillin sensitive | 0.13 |
| S. aureus/methicillin resistant | 4.0 |
| S. aureus/quinolone resistant | 4.0 |
| S. epidermidis/methicillin sensitive | 0.25 |
| S. epidermidis/methicillin resistant | 4.0 |
| S. pneumoniae/penicillin sensitive | 0.25 |
| S. pneumoniae/penicillin resistant | 0.25 |
| P. aeruginosa | 8.0 |
| H. influenzae/β-lactamase positive | 0.06 |
| H influenzae/βlactamase negative | 0.06 |

All of the foregoing concentrations are expressed as micrograms per milliliter ("mcg/ml").

The appropriate antibiotic concentration for ophthalmic compositions will generally be an amount of the antibiotic sufficient to provide a concentration in the aqueous humor and lacrimal fluid of the eye equal to or greater than the MIC90 level for the antibiotic, relative to gram-negative and gram-positive organisms commonly associated with ophthalmic infections. Such amounts are referred to herein as "an antimicrobial effective amount". The compositions of the present invention contain Moxifloxacin or a pharmaceutically useful hydrate or salt thereof in a concentration of 0.1 to 1.0 percent by weight ("wt. %") of the compositions.

The compositions of the present invention may also contain one or more anti-inflammatory agents. The anti-inflammatory agents utilized in the present invention are broadly classified as steroidal or non-steroidal. The preferred steroidal anti-inflammatory agents are glucocorticoids.

The preferred glucocorticoids include dexamethasone, loteprednol, rimexolone, prednisolone, fluorometholone, hydrocortisone mometasone, fluticasone, beclomethasone, flunisolide, triamcinolone and budesonide.

The dexamethasone derivatives described in U.S. Patent No. 5,223,493 (Boltralik) are also preferred steroidal anti-inflammatory agents, particularly with respect to compositions for treating ophthalmic inflammation. The following compounds are especially preferred: 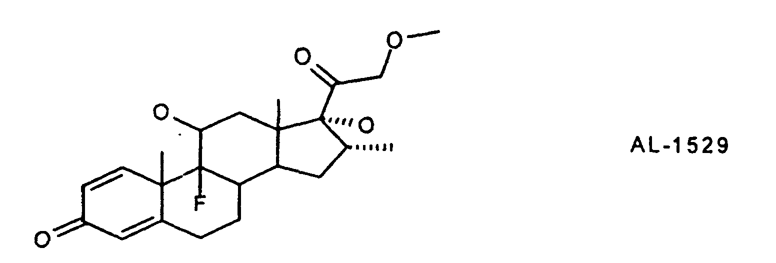 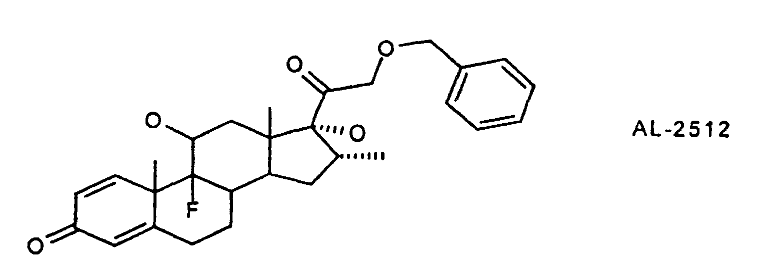 These compounds are referred to herein as "21-ether derivatives of dexamethasone". The 21-benzyl ether derivative (i.e., compound AL-2512) is particularly preferred.

The preferred non-steroidal anti-inflammatory agents are: prostaglandin H synthetase inhibitors (Cox I or Cox II), also referred to as cyclooxygenase type I and type II inhibitors, such as diclofenac, flurbiprofen, ketorolac, suprofen, nepafenac, amfenac, indomethacin, naproxen, ibuprofen, bromfenac, ketoprofen, meclofenamate, piroxicam, sulindac, mefanamic acid, diflusinal, oxaprozin, tolmetin, fenoprofen, benoxaprofen, nabumetome, etodolac, phenylbutazone, aspirin, oxyphenbutazone, NCX-4016, HCT-1026, NCX-284, NCX-456, tenoxicam and carprofen; cyclooxygenase type II selective inhibitors, such as NS-398, vioxx, celecoxib, P54, etodolac, L-804600 and S-33516; PAF antagonists, such as SR-27417, A-137491, ABT-299, apafant, bepafant, minopafant, E-6123, BN-50727, nupafant and modipafant; PDE IV inhibitors, such as ariflo, torbafylline, rolipram, filaminast, piclamilast, cipamfylline, CG-1088, V-1 1294A, CT-2820, PD-168787, CP-293121, DWP-205297, CP-220629, SH-636, BAY-19-8004, and roflumilast; inhibitors of cytokine production, such as inhibitors of the NFkB transcription factor; or other anti-inflammatory agents known to those skilled in the art.

The concentrations of the anti-inflammatory agents contained in the compositions of the present invention will vary based on the agent or agents selected and the type of inflammation being treated. The concentrations will be sufficient to reduce inflammation in the targeted ophthalmic tissues following topical application of the compositions to those tissues. Such an amount is referred to herein as "an anti-inflammatory effective amount". The compositions of the present invention will typically contain one or more anti-inflammatory agents in an amount of from 0.01 to 1.0 wt.%.

The compositions are typically administered to the affected ophthalmic tissues by topically applying one to four drops of a sterile solution or suspension, or a comparable amount of an ointment, gel or other solid or semisolid composition, one to four times per day. However, the compositions may also be formulated as irrigating solutions that are applied to the affected ophthalmic tissues during surgical procedures.

The ophthalmic compositions of the present invention will contain Moxifloxacin or a pharmaceutically useful hydrate or salt thereof and preferably one or more anti-inflammatory agents, in pharmaceutically acceptable vehicles. The compositions will typically have a pH in the range of 4.5 to 8.0. The ophthalmic compositions must also be formulated to have osmotic values that are compatible with the aqueous humor of the eye and ophthalmic tissues. Such osmotic values will generally be in the range of from 200 to 400 milliosmoles per kilogram of water ("mOsm/kg"), but will preferably be about 300 mOsm/kg.

Ophthalmic pharmaceutical products are typically packaged in multidose form. Preservatives are thus required to prevent microbial contamination during use. Suitable preservatives include: polyquaternium-1, benzalkonium chloride, thimerosal, chlorobutanol, methyl paraben, propyl paraben, phenylethyl alcohol, edetate disodium, sorbic acid, or other agents known to those skilled in the art. The use of polyquatemium-1 as the antimicrobial preservative is preferred. Typically such preservatives are employed at a level of from 0.001% to 1.0% by weight.

The solubility of the components of the present compositions may be enhanced by a surfactant or other appropriate co-solvent in the composition. Such co-solvents include polysorbate 20, 60, and 80, polyoxyethylene/polyoxypropylene surfactants (e.g., Pluronic F-68, F-84 and P-103), cyclodextrin, or other agents known to those skilled in the art. Typically such co-solvents are employed at a level of from 0.01% to 2% by weight.

The use of viscosity enhancing agents to provide the compositions of the invention with viscosities greater than the viscosity of simple aqueous solutions may be desirable to increase ocular absorption of the active compounds by the target tissues or increase the retention time in the eye. Such viscosity building agents include, for example, polyvinyl alcohol, polyvinyl pyrrolidone, methyl cellulose, hydroxy propyl methylcellulose, hydroxyethyl cellulose, carboxymethyl cellulose, hydroxy propyl cellulose or other agents know to those skilled in the art. Such agents are typically employed at a level of from 0.01% to 2% by weight.

The following examples are provided to further illustrate the ophthalmic compositions of the present invention.

### Example 1

| **Ophthalmic Solution** | |
|---|---|
| **Ingredient** | **Amount (wt. %)** |
| Moxifloxacin | 0.35 |
| Sodium Acetate | 0.03 |
| Acetic Acid | 0.04 |
| Mannitol | 4.60 |
| EDTA | 0.05 |
| Benzalkonium Chloride | 0.006 |
| Water | q.s. 100 |

### Example 2

| **Ophthalmic Suspension** | |
|---|---|
| **Ingredient** | **Amount (wt. %)** |
| Moxifloxacin | 0.3 |
| Dexamethasone, Micronized USP | 0.10 |
| Benzalkonium Chloride | 0.01 |
| Edetate Disodium, USP | 0.01 |
| Sodium Chloride, USP | 0.3 |
| Sodium Sulfate, USP | 1.2 |
| Tyloxapol, USP | 0.05 |
| Hydroxyethylcellulose | 0.25 |
| Sulfuric Acid and/or Sodium Hydroxide, NF | q.s. for pH adjustment to 5.5 |
| Purified Water, USP | q.s. to 100 |

### Example 3

| **Ophthalmic Ointment** | |
|---|---|
| **Ingredient** | **Amount (wt.%)** |
| Moxifloxacin | 0.35 |
| Mineral Oil, USP | 2.0 |
| White petrolatium, USP | q.s 100 |

### Example 4

| **Ophthalmic Ointment** | |
|---|---|
| **Ingredient** | **Amount (wt.%)** |
| Moxifloxacin | 0.3 |
| Fluorometholone Acetate, USP | 0.1 |
| Chlorobutanol, Anhydrous, NF | 0.5 |
| Mineral Oil,USP | 5 |
| White Petrolatum, USP | q.s. 100 |

The invention has been described herein by reference to certain preferred embodiments. However, as obvious variations thereon will become apparent to those skilled in the art, the invention is not to be considered as limited thereto.

## Claims

1. A topical ophthalmic pharmaceutical composition comprising moxifloxacin or a pharmaceutically useful hydrate or salt thereof in a concentration of 0.1 to 1.0 wt. % and a pharmaceutical acceptable vehicle therefor.

2. A topical composition according to Claim 1, wherein the composition further comprises an anti-inflammatory effective amount of a steroidal or non-steroidal anti-inflammatory agent.

3. A topical composition according to Claim 2, wherein the anti-inflammatory agent comprises a glucocorticoid.

4. A topical composition according to Claim 3, wherein the glucocorticoid is selected from the group consisting of dexamethasone, rimexolone, prednisolone, fluorometholone, hydrocortisone, mometasone, fluticasone, beclomethasone, flunisolide, triamcinolone and budesonide.

5. A topical composition according to Claim 4, wherein the anti-inflammatory agent comprises dexamethasone.

6. A topical compsition according to claim 5 wherein the anti-inflammatory agent comprises a 21-ether derivative of dexamethasone.

7. A topical composition according to claim 6 wherein the anti-inflammatory agent comprises a 21-benzyl ether derivative of dexamethasone.

8. A topical composition according to Claim 2, wherein the anti-inflammatory agent comprises a non-steroidal agent selected from the group consisting of prostaglandin H synthetase inhibitors, PAF antagonists, and PDE IV inhibitors.

9. A topical composition according to claim 8 wherein the anti-inflammatory agent comprises nepafenac.

10. A topical composition according to claim 8 wherein the anti-inflammatory agent comprises ketorolac.

11. A topical composition according to claim 8 wherein the anti-inflammatory agent comprises diclofenac.

12. Use of moxifloxacin or a pharmaceutically useful hydrate or salt thereof for the preparation of a topical composition comprising 0.1 to 1.0 wt. % of moxifloxacin for treating or preventing ophthalmic infections.

13. Use according to Claim 12, wherein the composition is for treating or preventing a condition selected from the group consisting of conjunctivitis, keratitis, blepharitis, dacyrocystitis, hordeolum and corneal ulceration.

14. Use according to Claim 12, wherein the composition is for application to the eye of a patient in connection with an ophthalmic surgical procedure.

15. Use according to Claim 12, wherein moxifloxacin or a pharmaceutical acceptable hydrate or salt thereof is used in combination with a steroidal or non steroidal anti-inflammatory agent.

16. Use according to Claim 15, wherein the anti-inflammatory agent comprises a glucocorticoid.

17. Use according to Claim 16, wherein the glucocorticoid is selected from the group consisting of dexamethasone, rimexolone, prednisolone, fluorometholone, hydrocortisone, mometasone, fluticasone, beclomethasone, flunisolide, triamcinolone and budesonide.

18. Use according to Claim 17, wherein the anti-inflammatory agent comprises dexamethasone.

19. Use according to Claim 18, wherein the anti-inflammatory agent comprises a 21-ether derivative of dexamethasone.

20. Use according to Claim 19, wherein the anti-inflammatory agent comprises a 21-benzyl ether derivative of dexamethasone.

21. Use according to Claim 15, wherein the anti-inflammatory agent comprises a non-steroidal agent selected from the group consisting of prostaglandin H synthetase inhibitors, PAF antagonists, and PDE IV inhibitors.

22. Use according to Claim 21, wherein the anti-inflammatory agent comprises nepafenac.

23. Use according to Claim 21, wherein the anti-inflammatory agent comprises ketorolac.

24. Use according to Claim 21, wherein the anti-inflammatory agent comprises diclofenac.

## Patentansprüche

1. Topische ophthalmische pharmazeutische Zusammensetzung enthaltend Moxifloxacin oder ein pharmazeutisch nützliches Hydrat oder Salz davon in einer Konzentration von 0,1 bis 1,0 Gew.-% und einen pharmazeutisch annehmbaren Träger dafür.

2. Topische Zusammensetzung nach Anspruch 1, wobei die Zusammensetzung weiterhin eine entzündungshemmend wirksame Menge eines steroidalen oder nicht-steroidalen entzündungshemmenden Mittels enthält.

3. Topische Zusammensetzung nach Anspruch 2, wobei das entzündungshemmende Mittel ein Glucocorticoid enthält.

4. Topische Zusammensetzung nach Anspruch 3, wobei das Glucocorticoid ausgewählt ist aus der Gruppe bestehend aus Dexamethason, Rimexolon, Prednisolon, Fluormetholon, Hydrocortison, Mometason, Fluticason, Beclomethason, Flunisolid, Triamcinolon und Budesonid.

5. Topische Zusammensetzung nach Anspruch 4, wobei das entzündungshemmende Mittel Dexamethason enthält.

6. Topische Zusammensetzung nach Anspruch 5, wobei das entzündungshemmende Mittel ein 21-Etherderivat von Dexamethason enthält.

7. Topische Zusammensetzung nach Anspruch 6, wobei das entzündungshemmende Mittel ein 21-Benzyletherderivat von Dexamethason enthält.

8. Topische Zusammensetzung nach Anspruch 2, wobei das entzündungshemmende Mittel ein nicht-steroidales Mittel enthält ausgewählt aus der Gruppe bestehend aus Prostaglandin-H-Synthetaseinhibitoren, PAF-Antagonisten und PDE-IV-Inhibitoren.

9. Topische Zusammensetzung nach Anspruch 8, wobei das entzündungshemmende Mittel Nepafenac enthält.

10. Topische Zusammensetzung nach Anspruch 8, wobei das entzündungshemmende Mittel Ketorolac enthält.

11. Topische Zusammensetzung nach Anspruch 8, wobei das entzündungshemmende Mittel Diclofenac enthält.

12. Verwendung von Moxifloxacin oder einem pharmazeutisch nützlichen Hydrat oder Salz davon zur Herstellung einer topischen Zusammensetzung mit 0,1 bis 1,0 Gew.-% Moxifloxacin zur Behandlung oder Verhütung von Augeninfektionen.

13. Verwendung nach Anspruch 12, wobei die Zusammensetzung zur Behandlung oder Verhütung eines Zustandes ausgewählt aus der Gruppe bestehend aus Konjunctivitis, Keratitis, Blepharitis, Dakryocystitis, Gerstenkorn und Hornhautgeschwürbildung vorgesehen ist.

14. Verwendung nach Anspruch 12, wobei die Zusammensetzung vorgesehen ist zum Auftragen auf das Auge eines Patienten im Zusammenhang mit einem ophthalmischen chirurgischen Verfahren.

15. Verwendung nach Anspruch 12, wobei Moxifloxacin oder ein pharmazeutisch annehmbares Hydrat oder Salz davon in Kombination mit einem steroidalen oder nicht-steroidalen entzündungshemmenden Mittel verwendet wird.

16. Verwendung nach Anspruch 15, wobei das entzündungshemmende Mittel ein Glucocorticoid enthält.

17. Verwendung nach Anspruch 16, wobei das Glucocorticoid ausgewählt ist aus der Gruppe bestehend aus Dexamethason, Rimexolon, Prednisolon, Fluormetholon, Hydrocortison, Mometason, Fluticason, Beclomethason, Flunisolid, Triamcinolon und Budesonid.

18. Verwendung nach Anspruch 17, wobei das entzündungshemmende Mittel Dexamethason enthält.

19. Verwendung nach Anspruch 18, wobei das entzündungshemmende Mittel ein 21-Etherderivat von Dexamethason enthält.

20. Verwendung nach Anspruch 19, wobei das entzündungshemmende Mittel ein 21-Benzyletherderivat von Dexamethason enthält.

21. Verwendung nach Anspruch 15, wobei das entzündungshemmende Mittel ein nicht-steroidales Mittel enthält ausgewählt aus der Gruppe bestehend aus Prostaglandin-H-Synthetaseinhibitoren, PAF-Antagonisten und PDE-IV-Inhibitoren.

22. Verwendung nach Anspruch 21, wobei das entzündungshemmende Mittel Nepafenac enthält.

23. Verwendung nach Anspruch 21, wobei das entzündungshemmende Mittel Ketorolac enthält.

24. Verwendung nach Anspruch 21, wobei das entzündungshemmende Mittel Diclofenac enthält.

## Revendications

1. Composition pharmaceutique ophtalmique topique comprenant de la moxifloxacine ou un hydrate ou sel pharmaceutiquement actif de celle-ci en une concentration de 0,1 à 1,0 % en poids et un véhicule pharmaceutique acceptable.

2. Composition topique suivant la revendication 1, dans laquelle la composition comprend de plus une quantité anti-inflammatoire efficace d'un agent anti-inflammatoire stéroïdien ou non stéroïdien.

3. Composition topique suivant la revendication 2, dans laquelle l'agent anti-inflammatoire comprend un glucocorticoïde.

4. Composition topique suivant la revendication 3, dans laquelle le glucocorticoïde est choisi dans le groupe comprenant la dexaméthasone, la rimexolone, la prednisolone, la fluorométholone, l'hydrocortisone, la mométasone, la fluticasone, la béclométhasone, le flunisolide, la triamcinolone et le budénoside.

5. Composition topique suivant la revendication 4, dans laquelle l'agent anti-inflammatoire comprend de la dexaméthasone.

6. Composition topique suivant la revendication 5, dans laquelle l'agent anti-inflammatoire comprend un dérivé 21-éther de la dexaméthasone.

7. Composition topique suivant la revendication 6, dans laquelle l'agent anti-inflammatoire comprend un dérivé 21-éther benzylique de la dexaméthasone.

8. Composition topique suivant la revendication 2, dans laquelle l'agent anti-inflammatoire comprend un agent non stéroïdien choisi dans le groupe comprenant les inhibiteurs de la prostaglandine H synthétase, les antagonistes de PAF et les inhibiteurs de PDE IV.

9. Composition topique suivant la revendication 8, dans laquelle l'agent anti-inflammatoire comprend du népafénac.

10. Composition topique suivant la revendication 8, dans laquelle l'agent anti-inflammatoire comprend du kétorolac.

11. Composition topique suivant la revendication 8, dans laquelle l'agent anti-inflammatoire comprend du diclofénac.

12. Utilisation de moxifloxacine ou d'un hydrate ou sel pharmaceutiquement actif de celle-ci pour la préparation d'une composition topique comprenant 0,1 à 1,0 % en poids de moxifloxacine pour le traitement ou la prévention d'infections ophtalmiques.

13. Utilisation suivant la revendication 12, dans laquelle la composition est utilisée pour le traitement ou la prévention d'un état choisi dans le groupe comprenant la conjonctivite, la kératite, la blépharite, la dacyrocystite, l'orgelet et l'ulcération cornéenne.

14. Utilisation suivant la revendication 12, dans laquelle la composition est utilisée pour une application à l'oeil d'un patient conjointement à un processus chirurgical ophtalmique.

15. Utilisation suivant la revendication 12, dans laquelle la moxifloxacine ou un hydrate ou sel pharmaceutique acceptable de celle-ci est utilisé en combinaison avec un agent anti-inflammatoire stéroïdien ou non stéroïdien.

16. Utilisation suivant la revendication 15, dans laquelle l'agent anti-inflammatoire comprend un glucocorticoïde.

17. Utilisation suivant la revendication 16, dans laquelle le glucocorticoïde est choisi dans le groupe comprenant la dexaméthasone, la rimexolone, la prednisolone, la fluorométholone, l'hydrocortisone, la mométasone, la fluticasone, la béclométhasone, le flunisolide, la triamcinolone et le budénoside.

18. Utilisation suivant la revendication 17, dans laquelle l'agent anti-inflammatoire comprend de la dexaméthasone.

19. Utilisation suivant la revendication 18, dans laquelle l'agent anti-inflammatoire comprend un dérivé 21-éther de la dexaméthasone.

20. Utilisation suivant la revendication 19, dans laquelle l'agent anti-inflammatoire comprend un dérivé 21-éther benzylique de la dexaméthasone.

21. Utilisation suivant la revendication 15, dans laquelle l'agent anti-inflammatoire comprend un agent non stéroïdien choisi dans le groupe comprenant les inhibiteurs de la prostaglandine H synthétase, les antagonistes de PAF et les inhibiteurs de PDE IV.

22. Utilisation suivant la revendication 21, dans laquelle l'agent anti-inflammatoire comprend du népafénac.

23. Utilisation suivant la revendication 21, dans laquelle l'agent anti-inflammatoire comprend du kétorolac.

24. Utilisation suivant la revendication 21, dans laquelle l'agent anti-inflammatoire comprend du diclofénac.
